(19) Europäisches Patentamt
European Patent Office
Office européen des brevets

(11) EP 1 169 648 B1

(12) EUROPEAN PATENT SPECIFICATION

(45) Date of publication and mention of the grant of the patent:
**24.05.2006 Bulletin 2006/21**

(21) Application number: **00918228.8**

(22) Date of filing: **21.03.2000**

(51) Int Cl.:
***G01N 33/68*** *(2006.01)* ***G01N 33/94*** *(2006.01)*
***G01N 33/58*** *(2006.01)* ***C07B 61/00*** *(2006.01)*
***G01N 33/50*** *(2006.01)*

(86) International application number:
**PCT/US2000/007524**

(87) International publication number:
**WO 2000/062074 (19.10.2000 Gazette 2000/42)**

# (54) USE OF 13C-NMR TO DETECT BINDING

VERWENDUNG VON 13C-NMR ZUM NACHWEIS VON BINDUNGEN

UTILISATION D'IMAGERIE PAR RESONANCE MAGNETIQUE 13C POUR LA DETECTION DE LIAISONS

(84) Designated Contracting States:
**AT BE CH CY DE DK ES FI FR GB GR IE IT LI LU MC NL PT SE**

(30) Priority: **09.04.1999 US 288924**

(43) Date of publication of application:
**09.01.2002 Bulletin 2002/02**

(73) Proprietor: **ABBOTT LABORATORIES**
**Abbott Park,**
**IL 60064-6050 (US)**

(72) Inventors:
- **FESIK, Stephen, W.**
  **Gurnee, IL 60031 (US)**
- **HAJDUK, Philip, J.**
  **Mundelein, IL 60060 (US)**

(74) Representative: **Modiano, Micaela Nadia**
**Modiano, Josif, Pisanty & Staub Ltd.,**
**Baaderstrasse 3**
**80469 München (DE)**

(56) References cited:
**WO-A-97/14814** **WO-A-97/18471**

- **CHEMICAL ABSTRACTS, vol. 132, Columbus, Ohio, US; abstract no. 175290, XP002142945 & A. MEDEK ET AL.: "The Use of differential chemical shifts for determining the binding site location and orientation of protein-bound ligands" JOURNAL OF THE AMERICAN CHEMICAL SOCIETY , vol. 122, no. 6, 1 June 2000 (2000-06-01), pages 1241-1242, Washington DC USA**
- **FEJZO J. ET AL PROTEIN SCIENCE vol. 5, no. 9, 1996, pages 1917 - 1921**
- **DATABASE MERRIAM-WEBSTER ONLINE**
- **FESIK S.W. ET AL J.AM.CHEM.SOC vol. 113, 1991, pages 7080 - 7081**
- **GARDNER K.H. ET AL J.AM.CHEM.SOC. vol. 119, 1997, pages 7599 - 7600**
- **GARDNER K.H. ET AL J.AM.CHEM.SOC. vol. 120, 1998, pages 11738 - 11748**
- **GOTO N.K. ET AL JOURNAL OF BIOMOLECULAR NMR vol. 13, April 1999, pages 369 - 374**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> Abbott Laboratories
Fesik, S. W.
Hajduk, P. J.

<120> USE OF 13C NUCLEAR MAGNETIC RESONANCE TO DETECT BINDING TO TARGET BIOMOLECULES

<130> 5816.US.P3

<140> US 09/288,924
<141> 1999-04-09

<150> US 09/241,184
<151> 1999-02-01

<150> US 08/744,701
<151> 1996-10-31

<150> US 08/678,903
<151> 1996-07-12

<150> US 08/558,633
<151> 1995-11-04

<160> 5

<170> FastSEQ for Windows Version 3.0

<210> 1
<211> 174
<212> PRT
<213> Artificial Sequence

<220>
<223> Catalytic Region of Human Stromelysin

<400> 1

```
Phe Arg Thr Phe Pro Gly Ile Pro Lys Trp Arg Lys Thr His Leu Thr
 1               5                  10                  15
Tyr Arg Ile Val Asn Tyr Thr Pro Asp Leu Pro Lys Asp Ala Val Asp
            20                  25                  30
Ser Ala Val Glu Lys Ala Leu Lys Val Trp Glu Glu Val Thr Pro Leu
        35                  40                  45
Thr Phe Ser Arg Leu Tyr Glu Gly Glu Ala Asp Ile Met Ile Ser Phe
    50                  55                  60
Ala Val Arg Glu His Gly Asp Phe Tyr Pro Phe Asp Gly Pro Gly Asn
65                  70                  75                  80
Val Leu Ala His Ala Tyr Ala Pro Gly Pro Gly Ile Asn Gly Asp Ala
                85                  90                  95
His Phe Asp Asp Asp Glu Gln Trp Thr Lys Asp Thr Thr Gly Thr Asn
            100                 105                 110
Leu Phe Leu Val Ala Ala His Glu Ile Gly His Ser Leu Gly Leu Phe
        115                 120                 125
```

```
His Ser Ala Asn Thr Glu Ala Leu Met Tyr Pro Leu Tyr His Ser Leu
    130                 135                 140
Thr Asp Leu Thr Arg Phe Arg Leu Ser Gln Asp Asp Ile Asn Gly Ile
145                 150                 155                 160
Gln Ser Leu Tyr Gly Pro Pro Pro Asp Ser Pro Glu Thr Pro
                165                 170
```

<210> 2
<211> 18
<212> DNA
<213> Artificial Sequence

<220>
<223> Primer

<400> 2
gaaatgaaga gtcttcaa 18

<210> 3
<211> 18
<212> DNA
<213> Artificial Sequence

<220>
<223> Primer

<400> 3
gcgtcccagg ttctggag 18

<210> 4
<211> 27
<212> DNA
<213> Artificial Sequence

<220>
<223> Primer

<400> 4

taccatggcc tatccattgg atggagc 27

<210> 5
<211> 30
<212> DNA
<213> Artificial Sequence

<220>
<223> Primer

<400> 5
ataggatcct taggtctcag gggagtcagg 30

**Description**

## Field of the Invention

The present invention relates to the use of nuclear magnetic resonance to detect binding between a putative ligand and a pre-selected $^{13}C$-enriched target molecules.

## Background of the Invention

One of the most powerful tools for discovering new drug leads is random screening of synthetic and natural product libraries to discover compounds that bind to a particular target molecule (for example, the identification of ligands to that target). Using this method, ligands may be identified by their ability to form a physical association with a target molecule or by their ability to alter a function of a target molecule.

When physical binding is sought, a target molecule is typically exposed to one or more compounds suspected of being ligands and assays are performed to determine if complexes between the target molecule and one or more of those compounds are formed. Such assays, as is well known in the art, test for gross changes in the target molecule (for example, changes in size, charge, mobility) that indicate complex formation.

Where functional changes are measured, assay conditions are established that allow for measurement of a biological or chemical event related to the target molecule (for example, an enzyme-catalyzed reaction, receptor-mediated enzyme activation, and the like). To identify an alteration, the function of the target molecule is determined before and after exposure to the test compounds.

Existing physical and functional assays have been used successfully to identify new drug leads for use in designing therapeutic compounds. There are, however, limitations inherent to those assays that compromise their accuracy, reliability and efficiency. A major problem with existing assays, for example, relates to the generation of "false positives". In a typical functional assay, a "false positive" result is generated for a compound that triggers the assay but which compound is not effective in eliciting the desired physiological response. In a typical physical assay, a "false positive" is a compound that, for example, attaches itself to the target but in a nonspecific manner (for example, non-specific binding). False positives are particularly prevalent and problematic when screening higher concentrations of putative ligands because many compounds have non-specific effects at those concentrations.

In a similar fashion, existing assays are frequently plagued by the problem of "false negatives", which result when a compound gives a negative response in the assay but, as found subsequently by some other method, is actually a ligand for the target. False negative results typically occur in assays that use concentrations of test compounds that are either too high (resulting in toxicity) or too low, relative to the binding or dissociation constant of the compound to the target.

Another problem with existing assays is the limited amount of information that is provided by the assay itself. While the assay may correctly identify compounds that attach to or elicit a response from the target molecule, those assays typically do not provide any information about either specific binding sites on the target molecule or structure activity relationships between the compound being tested and the target molecule. The inability to provide any such information is particularly problematic where the screening assay is being used to identify leads for further study.

It has recently been suggested that X-ray crystallography can be used to identify the binding sites of organic solvents on macromolecules. However, this method cannot determine the relative binding affinities at different sites on the target. It is only applicable to very stable target proteins that do not denature in the presence of high concentrations of organic solvents. In addition, due to the long time needed to determine the individual crystal structures, this approach is not a suitable method for rapidly testing a large number of compounds that are chemically diverse, but is limited to mapping the binding sites of only a few organic solvents.

Rapid, efficient, and reliable methods of determining ligand/target binding, and mapping binding sites on the target substance are disclosed in United States Patent Nos. 5,698,401 and 5,804,390, to Fesik, *et al.* These patents disclose methods of detecting binding of a ligand compound to a target biomolecule by generating first and second nuclear magnetic resonance correlation spectra from target biomolecules which have been isotopically enriched with the NMR-detectable $^{15}N$ nucleus. The first spectrum is generated from data collected on the target substance in the absence of ligands, and the second in the presence of one or more ligands. A comparison of the two spectra permits determination of which compounds in the mixture of putative ligands bind(s) to the target biomolecule, as well as specific information about the site of binding. Since the methods elicit information about the binding sites on the target molecule, the methods can be used for optimizing the design of ligands to a pre-selected target.

Because the NMR methods of Fesik, *et al.* , supra, require nitrogen-containing target substances due to the dependence of those methods on isotopic-enrichment of the target molecule with $^{15}N$, it would be a valuable contribution to the art to provide an alternative method which employs a different type of isotopically-enriched target molecule.

Fejzo J. et al.: Protein Science, vol 5, no 9, 1996, pages 1917 - 1921 discloses a method of detecting binding

between a putative ligand (calcineurin subunit B: CnB) and a pre-selected $^{13}C$ enriched target molecule (a FKBP. $^{12/13}C$ FK506 complex).

Fesik, S. W. et al.: J. Am. Chem. Soc., vol 113, 1991, pages 7080-7081 discloses a method of detecting binding between a putative ligand (cyclophilin) and a pre-selected 13C-enriched target molecule (cyclosporin A).

Gardner, K.H. et al.: J. Am. Chem. Soc., vol 119, 1997 pages 7599-7600 discloses method to generate uniformely $^{13}C$ labeled proteins via 13C-2H glucose source.

Gardner, K.H. et al.: J. Am. Chem. Soc. vol 120, 1998, pages 11738-11748 discloses a method of uniformely label proteins with $^{15}N$, $^{13}C$ and $^{2}H$ and the utility of samples labeled in this manner.

## Summary of the Invention

The instant invention provides a method of detecting binding between one or more putative ligands to a pre-selected, $^{13}C$-enriched target molecule.

The instant invention further provides a method of screening a mixture of compounds for binding to a pre-selected, $^{13}C$-enriched target molecule.

## Brief Description of the Drawings

Figure 1 illustrates a $^{13}C/^{1}H$ correlation spectrum of uniformly $^{13}C$-labeled FKBP.

Figure 2 illustrates the methyl regions of $^{13}C/^{1}H$ correlation spectra of uniformly $^{13}C$-labeled FKBP before (thin multiple contours) and after (thick single contours) addition of 2- phenylimidazole (0.12 mM).

Figure 3 illustrates the methyl regions of $^{13}C/^{1}H$ correlation spectra of FKBP selectively $^{13}C/^{15}N/^{2}H$-labeled at valinyl and leucyl residues before (thin multiple contours) and after (thick single contours) addition of 2-phenylimidazole (0.25 mM).

Figure 4 illustrates the methyl regions of $^{13}C/^{1}H$ correlation spectra of FKBP selectively $^{13}C$-labeled at alanyl residues before (thin multiple contours) and after (thick single contours) addition of 2-phenylimidazole (0.25 mM).

Figure 5 illustrates a "stick model" depiction of the three-dimensional structure of FKBP.

## Detailed Description of the Invention

Terms used throughout this specification have their usually accepted meanings. The following specific terms have the ascribed meanings.

"DTT" means dithiothreitol.
"FKBP" refers to FK-binding protein.
"HEPES" denotes N-2-hydroxyethylpiperazine-N'-2-ethylsulfonic acid.
"IPTG" means isopropyl-β-D-thiogalactopyranoside.
"PMSF" refers to α-toluenesulfonyl fluoride.
"SCD" refers to the catalytic domain (residues 81-256) of stromelysin.

Any target biomolecule which gives a high resolution NMR spectrum and which can be uniformly or selectively enriched with $^{13}C$ can be used in the methods of the present invention. These methods are thus applicable to any desired $^{13}C$-enriched target biomolecule, including lipoproteins, lipoprotein fragments, glycoproteins, glycoprotein fragments, proteins, protein fragments, polypeptides, DNA and RNA. The natural isotopic abundance of $^{13}C$ is 1.11%. Thus, the probability that any given carbon atom within an organic molecule is $^{13}C$ is normally about 0.0111.

In order to increase the strength of a NMR signal evincing data related to spin coupling between the nucleus of a $^{13}C$ carbon atom and any adjacent hydrogen atoms, it is desirable to increase the natural $^{13}C$ content of the target molecule being studied. This is accomplished by either uniformly or selectively enriching the target molecule with $^{13}C$. As used throughout this specification and the appended claims, the terms "uniform enrichment," "uniformly enriching," "uniformly enriched," uniform labeling" and "uniformly labeled" mean increasing to a value greater than 0.0111, by synthetic means, the probability that a carbon atom randomly selected throughout the target molecule will be $^{13}C$. On the other hand, the terms "specific enrichment," "specifically enriching," "specifically enriched," "specifically labeling" and "specifically labeled" mean increasing to a value greater than 0.0111, by synthetic means, the probability that carbon atoms at one or more specific pre-selected site(s) within the target molecule will be $^{13}C$.

For example, biomolecules expressed by genetically modified microorganisms grown in a nutrient medium containing uniformly $^{13}C$-enriched glucose will be uniformly $^{13}C$-enriched. On the other hand, a protein expressed by a

genetically modified microorganism in a nutrient medium containing alanine that is $^{13}C$-enriched only on the methyl side chain will be specifically enriched by $^{13}C$ at those alanyl residues contained within the expressed protein.

The method of the present invention, which employs isotopic enrichment with $^{13}C$, is applicable to any organic target molecule, including those containing nitrogen. The method also permits the analysis of target molecules in which specific carbon atom sites have been enriched (for example, methyl groups of alanyl, leucyl, isoleucyl, and valinyl residues). Methyl groups have favorable relaxation properties compared to amide groups, which is advantageous when applied to larger target biomolecules (MW > 30 kDa).

Polypeptides and proteins perform many pivotal roles in living organisms. The examples provided below employ polypeptides to illustrate the instant method. Polypeptides and protein fragments comprise preferred classes of target substances for the method of the present invention. However, it is to be understood that the method of the present invention is applicable to other target substances which can be $^{13}C$-enriched.

The preparation of uniformly and specifically $^{13}C$-enriched exemplary polypeptide target molecules is set forth below. One means of preparing adequate quantities of either uniformly or specifically $^{13}C$-enriched polypeptide-containing target molecules involves the transformation of a host cell with an expression vector containing a polynucleotide encoding the desired polypeptide. The protein or polypeptide protein fragment is expressed by culturing the transformed cell line in a medium containing assimilable sources of $^{13}C$ well known in the art. A preferred assimilable source for uniform $^{13}C$ labeling is uniformly $^{13}C$-labeled glucose or U-$^{13}C$-glucose, available from Cambridge Isotope Laboratories. For site-specific labeling, assimilable sources for $^{13}C$-labeling of a target polypeptide include commercially available specifically $^{13}C$-labeled amino acids. According to the present invention for specific enrichment, the assimilable sources of $^{13}C$ contained in the nutrient medium are $^{13}C$-labeled biosynthetic precursors of amino acids that consist of α-keto-butyrate which is the biosynthetic precursor of isoleucine and α-keto-isovalerate which is the biosynthetic precursor of both valine and leucine. Scheme I below shows how the specifically $^{13}C$-enriched biosynthetic precursors of leucine, isoleucine, and valine, can be synthesized. The comparatively inexpensive $^{13}C$-enriched methyl iodide ($H_3{}^{13}CI$) may be employed as the source for isotopic enrichment to produce C-terminally-labeled α-keto-butyric acid and α-keto-isovaleric acid.

The comparative use of a uniformly $^{13}C$-enriched nutrient such as $^{13}C$-enriched glucose is a convenient means of introducing $^{13}C$-enrichment into a target substance; however, it is very expensive. Furthermore, a vast majority of the carbon sites in uniformly $^{13}C$-labeled targets will have a covalently bonded neighbor which is also $^{13}C$-labeled, introducing $^{13}C$-$^{13}C$ coupling which can negatively impact both the signal-to-noise and relaxation properties of $^{13}C$-labeled sites in the target biomolecule. A particular advantage is achieved by site-specifically labeling the target polypeptide with $^{13}C$. As stated above, this can be accomplished by including commercially available $^{13}C$-labeled amino acids in the nutrient medium. This too, however, is a costly alternative, but may be desirable in some circumstances when labeling of certain types of aminoacyl residues in the target polypeptide is required. According to the present invention the method of $^{13}C$-labeling a polypeptide target molecule comprises growing the genetically modified cell line in a nutrient medium containing $^{13}C$-labeled biosynthetic precursors of amino acids that consist of 13C α-keto-butyric acid and $^{13}C$ α-keto-isovaleric acid. The biosynthetic products of these precursors are leucine, isoleucine, and valine in which particular side-chain methyl groups are $^{13}C$-enriched. Because the methyl groups each have three hydrogen atoms connected to a $^{13}C$-labeled carbon atom, the corresponding NMR signals are particularly strong and distinctive.

The synthetic sequence for labeled α-keto-butyric acid and α-keto-isovaleric acid involves the methylation of the terminal carbon atom in pyruvic acid with $^{13}C$ -enriched methyl iodide. Normally, the alkylation of α-keto acids, such as pyruvate, is inherently difficult and is accompanied by decomposition of the enolate intermediate with the formation of numerous side products. However, T. Spencer, *et al., Tetrahedron_Letters,* 1975, 3889, and D. R. Williams, *et* al., ibid, 1990, 5881, have shown that alkylation of the corresponding oxime can be easily accomplished, and D. Enders, *et al.*, *Angew. Chem. Int. Eng. Ed.,* 1992, 618 and D. Enders, *et al.*, *Synlett,* 1992, 901 have demonstrated that the hydrazone is also readily alkylated without decomposition.

In Scheme I, *tert*-butyl pyruvate, **1**, is converted to the corresponding N,N-dimethylhydrazone, **2**, by reaction with N,N-dimethylhydrazine in diethyl ether at room temperature. The resulting hydrazone, **2**, is cooled in tetrahydrofuran solution to -78°C, and treated with lithium bromide, followed by lithium diisopropylamide to form the intermediate aza-allyl enolate. The enolate is alkylated with $^{13}C$-labeled methyl iodide to produce hydrazone **3**. A second course of alkylation of **3** produces the labeled dimethylated hydrazone, **4**. Treatment of **3** and **4** with trifluoroacetic acid in methylene chloride at 0°C cleaves both the hydrazine and ester groups to produce the corresponding $^{13}C$-terminally labeled α-ketoacids, **5** and **6** (4($^{13}C$) α keto-butyric acid and 4-($^{13}C$)-3-($^{13}C$) α keto-methylbutyric acid, respectively).

## Scheme I

## Chemical Synthesis of Labeled Precursors

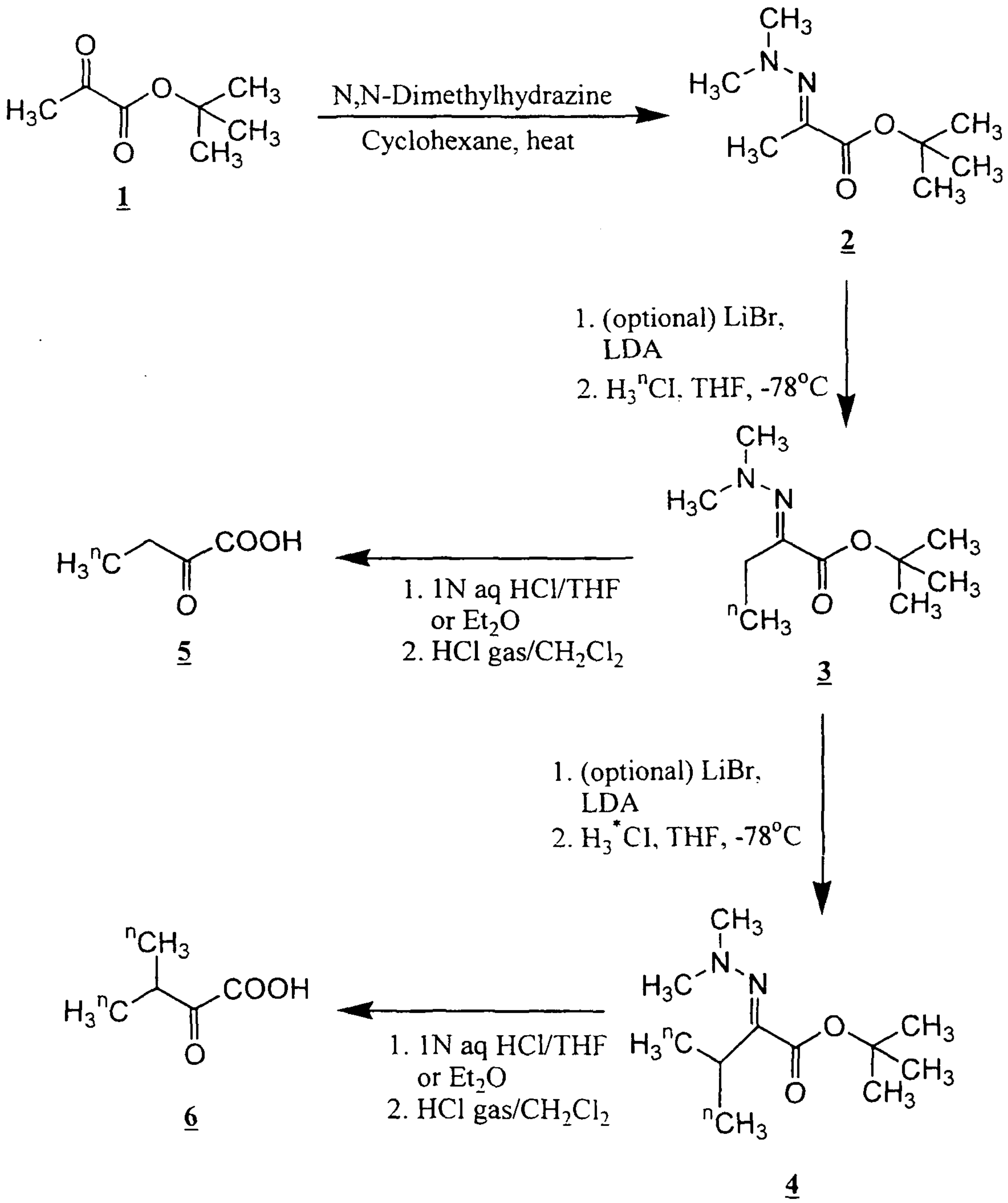


1 2 3 4 5 6

Reaction Schemes II, III, and IV illustrate, respectively, how these α-ketoacids are biosynthetically converted into $^{13}C$-leucine, $^{13}C$-isoleucine, and $^{13}C$-valine. In all of the Schemes, the sites of isotopic enrichment are indicated by asterisks.

## Scheme II

## Biochemical Synthesis of Labeled Isoleucine

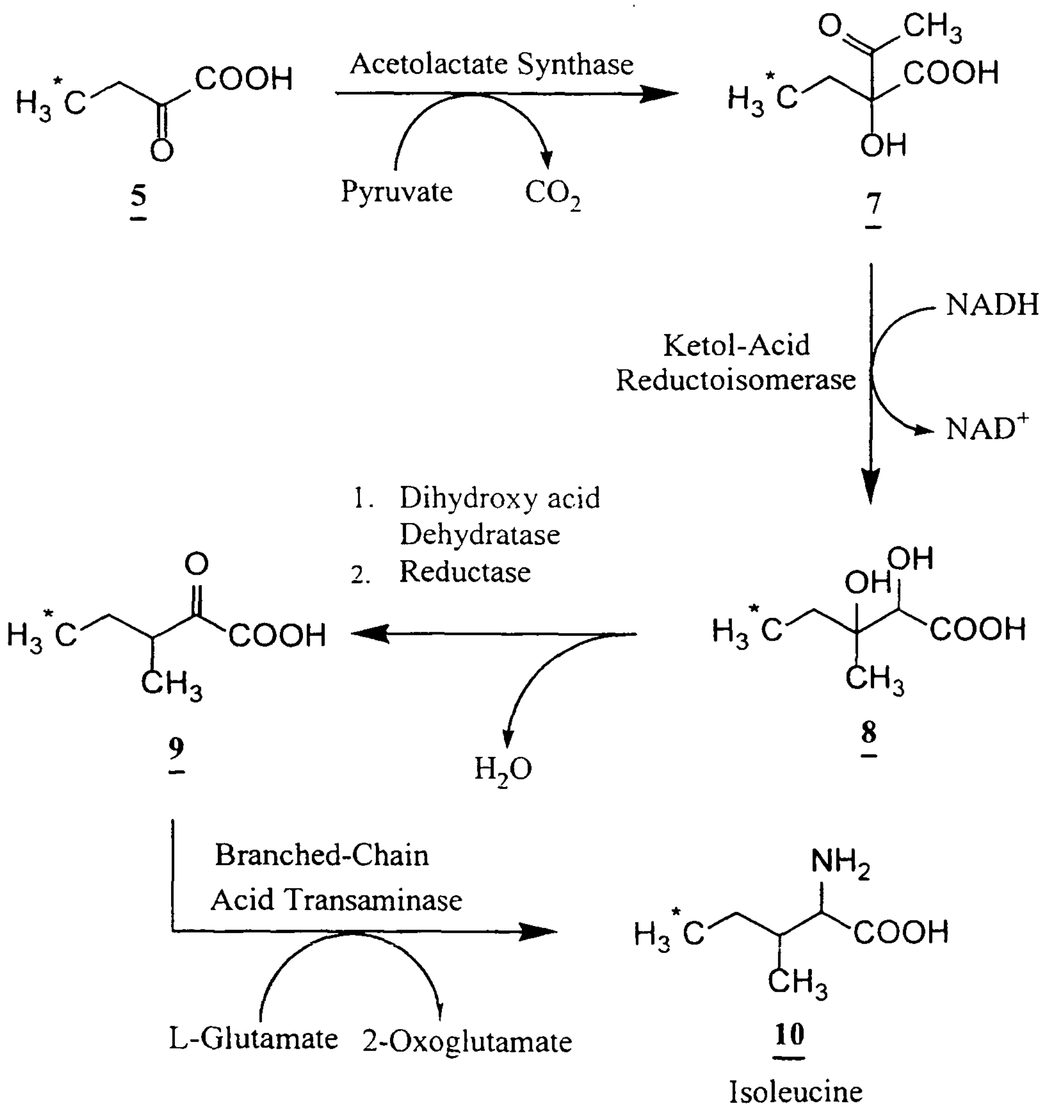


5

7

8

9

10

Isoleucine

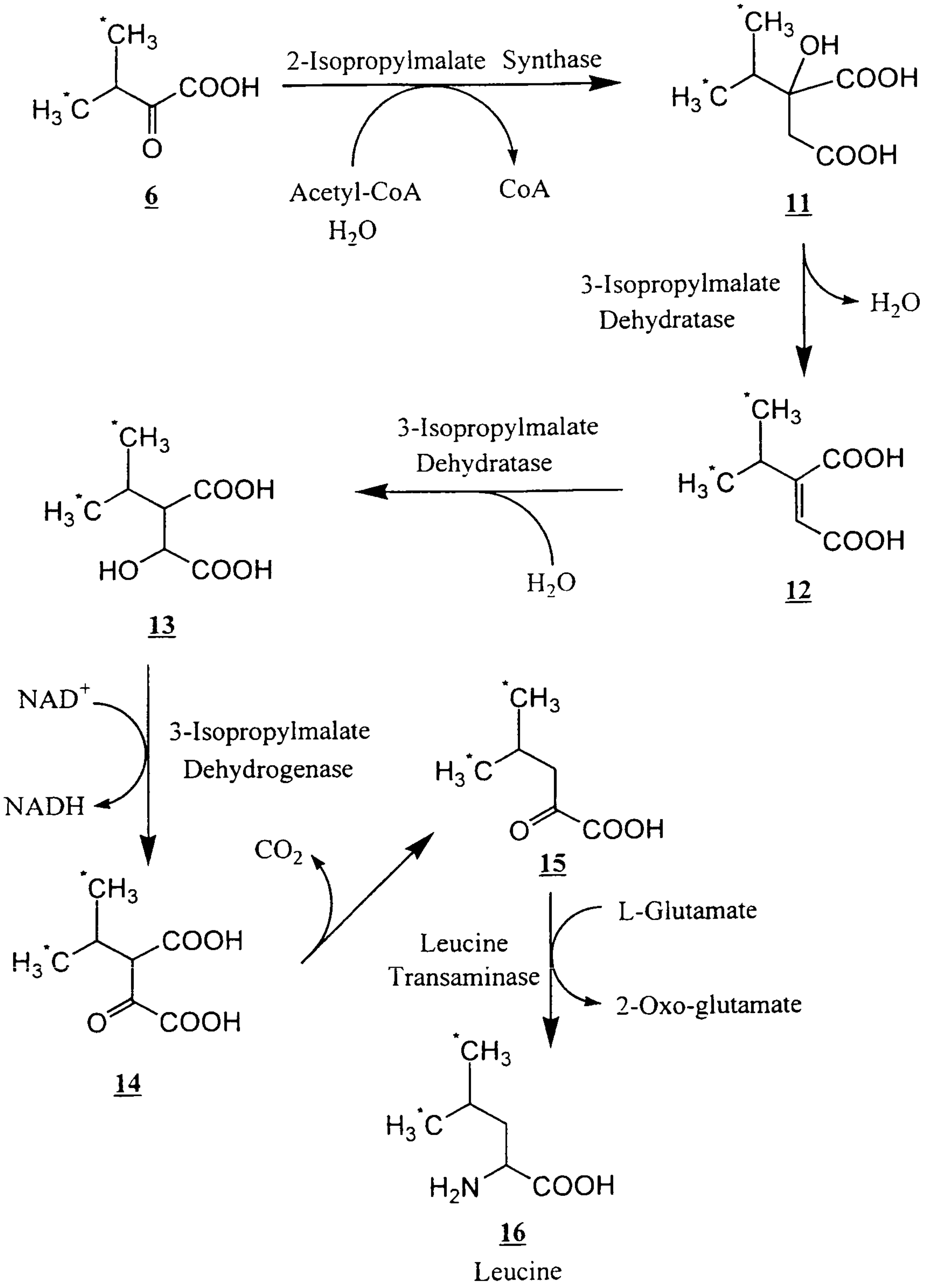


6

11

12

13

14

15

16

Leucine

Scheme IV

Biochemical Synthesis of Valine

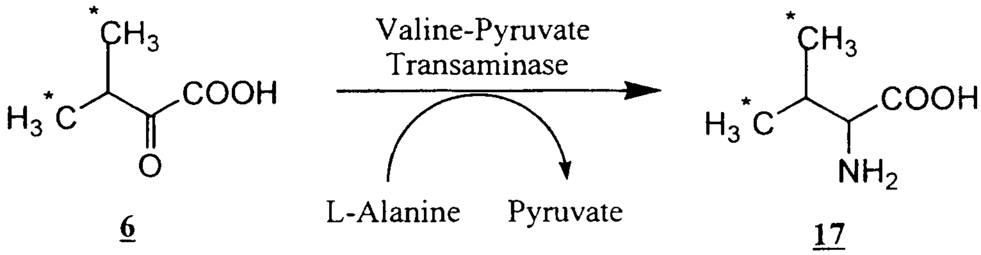


6

17

L-Valine

Means for preparing expression vectors that contain polynucleotide sequences coding specific polypeptides and for transforming host cells with those vectors are well known in the art. (See, for example, R. W. Old, et al., "Techniques of Gene Manipulation," Blackwell Science, London, 1994, and similar treatises in the field.) Likewise, methods for culturing the transformed cells to express the coded polypeptide and for isolating, purifying and re-folding the polypeptide are also well known in the art. Examples presented below describe the production from modified *E. coli* of $^{13}$C-enriched samples of the 81-256 amino acid catalytic region of human stromelysin and FK binding protein (FKBP), and the use of these isotopically-enriched polypeptides in the instant methods.

Where the method is employed to screen more than one compound for binding to the target molecule, for example a mixture or a library of compounds, and where a difference arises between the first spectrum generated from the target molecule alone and that generated from the target molecule in the presence of compound(s), additional steps are performed to identify which specific compound or compounds contained in the mixture is actually binding to the target molecule. Those additional steps include exposing the $^{13}$C-enriched target molecule individually to each compound of the mixture; generating a two-dimensional $^{13}$C/$^{1}$H NMR correlation spectrum of the labeled target molecule that has been individually exposed to each compound; and comparing each spectrum to the first spectrum generated from the target molecule alone to determine differences in any of those compared spectra. The differences in the spectra facilitate the identification of a compound that is a ligand.

The chemical shift values of the particular $^{13}$C/$^{1}$H signals in the two dimensional correlation spectrum correspond to known specific locations of atomic groupings in the target molecule (for example, the carbon atoms of a particular amino acid residue in the target molecule or, in the case of a polypeptide specifically labeled at the methyl groups of alanyl, leucyl, isoleucyl, and valinyl residues). The screening process of this invention thus allows not only for the identification of which compound(s) bind to a particular target molecule, but also permits the determination of the particular amino acids that are affected by the binding of the compound to the target molecule. The chemical shift values may reflect a change in the conformation of the target molecule, or may reflect the binding of the ligand compound at the site that corresponds to that particular signal.

In addition, the dissociation constant, $K_D$, for a given ligand and its target molecule can be determined by this process, if desired, by generating a first two-dimensional $^{13}$C /$^{1}$H NMR correlation spectrum of a $^{13}$C-labeled target molecule; exposing the labeled target molecule to various concentrations of a ligand; generating a two-dimensional $^{13}$C/$^{1}$H NMR correlation spectrum at each concentration of ligand employed; comparing the spectra generated to the first spectrum of the target molecule; and calculating the dissociation constant between the target molecule and the ligand from those differences using Equation 1:

$$K_D = \frac{([P_0] - x)([L_0] - x)}{x}$$

where $[P_0]$ is the total molar concentration of the target molecule, $[L_0]$ is the total molar concentration of the ligand, and x is the molar concentration of the bound species. The value of x is determined from the NMR chemical shift data

according to Equation 2:

$$x = \frac{\delta_{observed} - \delta_{free}}{\Delta}$$

where $\delta_{observed}$ is the observed chemical shift value, $\delta_{free}$ is the chemical shift value for the free species, and $\Delta$ is the difference between the limiting chemical shift value for saturation ($\delta_{saturation}$) and the chemical shift value of the target molecule free of bound ligand ($\delta_{free}$).

The dissociation constant is then determined by varying its value until a best fit is obtained with the observed data using standard curve-fitting statistical methods. In those situations where the value of $\delta_{saturation}$ is not directly known, $K_D$ and $\delta_{saturation}$ are varied and the resulting data subjected to the same curve-fitting statistical method.

An advantageous capability of the screening method is its ability to determine the dissociation constant of one ligand of the target molecule in the presence of a second molecule already bound to the ligand. This is generally not possible with other methods which employ "wet chemical" analytical methods of determining binding of a ligand to a target molecule substrate.

The process of determining the dissociation constant of a ligand can be performed in the presence of a second bound ligand. Accordingly, the $^{13}C$-labeled target molecule is bound to that second ligand before exposing that target to the test compounds. The screening method is additionally able to provide information regarding the binding of a second or subsequent ligand to the target molecule. This second ligand may be chemically linked to the first ligand bound to the target molecule, thus providing a new composite molecule for use in affecting the target molecule.

The screening method of the present invention begins with the generation or acquisition of a two-dimensional $^{13}C/^{1}H$ correlation spectrum of the isotopically enriched target molecule. As stated above, the target molecule can be either uniformly enriched with $^{13}C$, or it can be specifically enriched by the incorporation of $^{13}C$-methyl groups in alanyl, leucyl, valinyl, and isoleucyl residues. Means for generating two-dimensional $^{13}C/^{1}H$ correlation spectra are well known in the art. The NMR spectra that are typically recorded are two-dimensional $^{13}C/^{1}H$ heteronuclear single quantum correlation (HSQC) spectra, although other techniques known to those skilled in the art can be used. Because the $^{13}C/^{1}H$ signals corresponding to the protein are usually well resolved, the chemical shift changes for individual $^{13}C/^{1}H$ pairs can be readily monitored. A representative two-dimensional $^{13}C/^{1}H$ correlation spectrum of a $^{13}C$-labeled target polypeptide (FKBP) is shown in Figure 1. A representative two-dimensional $^{13}C/^{1}H$ correlation spectrum of a specifically $^{13}C$-enriched FKBP is shown in Figure 3.

Following exposure of the $^{13}C$-labeled target molecule to one or more test compounds, a second two-dimensional $^{13}C/^{1}H$ NMR correlation spectrum is generated. That spectrum is generated in the same manner as set forth above. The first and second spectra are then compared to determine whether there are any differences between the two spectra. Differences in the two-dimensional $^{13}C/^{1}H$ NMR correlation spectra that indicate the presence of a ligand correspond to $^{13}C$-labeled sites in the target molecule. Those differences are determined using standard procedures well known in the art. Figure 2 shows the methyl regions of uniformly $^{13}C$-labeled FKBP before (thin multiple contours) and after (thick single contours) addition of 2-phenylimidazole (0.12 mM).

Particular signals in a two-dimensional $^{13}C/^{1}H$ correlation spectrum correspond to specific carbon and proton atoms in the target molecule (for example, particular methyl groups of the amino acid residues in the protein). By way of example, it can be seen from Figure 3 that chemical shifts observed in two-dimensional $^{13}C/^{1}H$ correlation spectra of FKBP exposed to a test compound occurred at residue positions 97 (leucine) and 55 (valine). The region of the protein that is responsible for binding to the individual compounds is identified from the particular carbon and proton atom pairs that change upon the addition of compound.

## EXAMPLES

Preparations 1 and 2 are comparative examples.

### Preparation 1

### Preparation of Uniformly $^{13}C$-Enriched Catalytic Domain of Human Stromelysin (SCD)

The 81-256 fragment (SEQ ID NO: 1) of stromelysin (SCD) is prepared by inserting a plasmid which codes for the production of the protein fragment into an E. *coli* strain and growing the genetically-modified bacterial strain in a suitable culture medium. The protein fragment is isolated from the culture medium, purified, and subsequently used in

the two-dimensional NMR analysis of its affinity with test compounds in accordance with the method of this invention. The procedures for the preparation processes are described below.

Human skin fibroblasts (ATCC No. CRL 1507) are grown and induced using the procedure described by Clark *et al.*, *Archiv. Biochem. and Biophys.* 241: 36 (1985). Total RNA is isolated from 1 g of cells using a RNAgents® Total RNA Isolation System Kit (Promega Corp.) following the manufacturer's instructions. A 1 μg portion of the RNA is denatured by heating at 80°C for five minutes and then subjected to reverse transcriptase PCR using a GenAmp® RNA PCR kit (Applied Biosystems/Perkin-Elmer) following the manufacturer's instructions.

Nested PCR is performed using first primers (a) GAAATGAAGAGTCTTCAA (SEQ ID NO: 2) and (b) GCGTCCCAGGTTCTGGAG (SEQ ID NO. 3) and thirty-five cycles of 94°C, two minutes; 45° C, two minutes; and 72°C, three minutes. This is followed by re-amplification with internal primers (c) TACCATGGCCTATCCATTGGATGGAGC (SEQ ID NO: 4) and (d) ATAGGATCCTTAGGTCTCAGGGGA GTCAGG (SEQ ID NO: 5) using thirty cycles under the same conditions described immediately above to generate a DNA sequence coding for amino acid residues 1-256 of human stromelysin.

The PCR fragment is then cloned into PCR cloning vector pT7BIue® (Novagen, Inc.) according to the manufacturer's instructions. The resulting plasmid is cut with NcoI and BamHI and the stromelysin fragment is sub-cloned into the expression vector pET3d (Novagen, Inc.).

A mature stromelysin expression construct coding for amino acid residues 81-256 plus an initiating methionine aminoacyl residue is generated from the 1-256 expression construct by PCR amplification. The resulting PCR fragment is first cloned into the pT7BIue® vector (Novagen, Inc.) and then sub-cloned into the pET3d vector (Novagen, Inc.), using the manufacturer's instructions in the manner described above, to produce plasmid pETST-83-256. This final plasmid is identical to that described by Qi-Zhuang et al., *Biochemistry,* 31: 11231 (1992) with the exception that the present plasmid codes for a peptide sequence beginning two amino acids earlier, at position 81, in the sequence of human stromelysin. Plasmid pETST-83-256 is transformed into *E. coli* strain BL21(DE3)/pLysS (Novagen, Inc.) in accordance with the manufacturer's instructions to generate an expression strain, BL21(DE3)/pLysS/pETST-255-1.

A pre-culture medium is prepared by dissolving 1.698 g of $NaH_2PO_4$•$7H_2O$, 0.45 g of $KH_2PO_4$, 0.075 g NaCl, 0.150 g $NH_4Cl$, 0.3 g U-$^{13}$C-glucose, 300 μl of 1M aqueous $MgSO_4$ solution, and 15 mL of aqueous $CaCl_2$ solution in 150 ml of deionized water. The resulting solution of pre-culture medium is sterilized and transferred to a sterile 500 ml baffle flask. Immediately prior to inoculation of the pre-culture medium with the bacterial strain, 150 ml of a solution containing 34 mg/ml, of chloramphenicol in 100% ethanol and 1.5 ml of a solution containing 20 mg/ml of ampicillin is added to the flask contents. The flask contents are then inoculated with 1 ml of glycerol stock of genetically modified E. *coli* strain BL21 (DE3) / pLysS / pETST-255-1. The flask contents are shaken (225 rpm) at 37°C until an optical density of 0.65 is observed.

A fermentation nutrient medium is prepared by dissolving 113.28 g of $Na_2HPO_4$•7H2O, 30 g of $KH_2PO_4$, 5 g NaCl and 10 mL of 1% DF-60 antifoam agent in 9604 mL of deionized water. This solution is placed in a New Brunswick Scientific Micros Fermenter and sterilized at 121°C for 40 minutes. Immediately prior to inoculation of the fermentation medium, the following pre-sterilized components are added to the fermentation vessel contents: 100 ml of a 10% aqueous solution of $NH_4Cl$, 15 g of uniformly $^{13}$C-enriched glucose, 20 ml of an aqueous 1M solution of $MgSO_4$, 1 ml of an aqueous 1M $CaCl_2$ solution, 5 ml of an aqueous solution of thiamin hydrochloride (10 mg/ml), 10 ml of a solution containing 34 mg/ml of chloramphenicol in 100% ethanol, and 1.9 g of ampicillin dissolved in the chloramphcnicol solution. The pH of the resulting solution is adjusted to pH 7.00 by the addition of an aqueous solution of 4N $H_2SO_4$.

The pre-culture of *E. coli* strain BL21(DE3)/pLysS/pETST-255-1 from the shake flask scale procedure described above is added to the fermenter contents and cell growth is allowed to proceed until an optical density of 0.48 is achieved. During this process, the fermenter contents are automatically maintained at pH 7.0 by the addition of 4N $H_2SO_4$ or 4N KOH as needed. The dissolved oxygen content of the fermenter contents is maintained above 55% air saturation through a cascaded loop which increased agitation speed when the dissolved oxygen content dropped below 55%. Air is fed to the fermenter contents at 7 standard liters per minute (SLPM) and the culture temperature is maintained at 37°C throughout the process.

The cells are harvested by centrifugation at 17,000 x g for 10 minutes at 4°C and the resulting cell pellets are collected and stored at -85°C. The wet cell yield is 3.5 g/L. Analysis of the soluble and insoluble fractions of cell lysates by sodium dodecyl sulfate polyacrylamide gel electrophoresis (SDS-PAGE) reveals that approximately 50% of the stromelysin is found in the soluble phase.

The stromelysin fragment prepared as described above is purified employing a modification of the technique described by Ye, *et al., Biochemistry,* 31: 11231 (1992). The harvested cells are suspended in 20 mM Tris-HCl buffer (pH 8.0), sodium azide solution containing 1mM $MgCl_2$, 0.5 mM $ZnCl_2$, 25 units/ml of Benzonase® enzyme (Benzon Pharma), and an inhibitor mixture made up of 4-(2-aminoethyl)benzenesulfonyl fluoride ("AEBSF") Leupeptin® , Aprotinin® and Pepstatin® (all at concentrations of 1 mg/ml). AEBSF, Leupeptin® , Aprotinin® , and Pepstatin® are available from American International Chemical. The resulting mixture is gently stirred for one hour and then cooled to 4°C. The cells are then sonically disrupted using a 50% duty cycle. The resulting lysate is centrifuged at 14,000 rpm for 30 minutes

and the pellet of insoluble fraction frozen at -80°C for subsequent processing.

Solid ammonium sulfate is added to the supernatant to the point of 20% of saturation and the resulting solution loaded onto a 700 ml phenyl Sepharose fast flow ("Q-Sepharose FF) column (Pharmacia Biotech.). Prior to loading, the Sepharose column is equilibrated with 50 mM Tris-HCl buffer (pH 7.6 at 4°C), 5 mM CaC12, and 1M $(NH_4)_2SO_4$. The loaded column is eluted with a linear gradient of decreasing concentrations of aqueous $(NH_4)_2SO_4$ (from 1M down to OM) and increasing concentrations of aqueous $CaCl_2$ (from 5 mM to 20 mM) in Tris-HCl buffer at pH 7.6. The active fractions of eluate are collected and concentrated in an Amicon stirred cell (Amicon, Inc.). The concentrated sample is dialyzed overnight in the starting buffer used with the Q-Sepharose FF column, 50 mM Tris-HCl (pH 8.2 at 4°C) with 10 mM $CaCl_2$.

The dialyzed sample is then loaded on the Q-Sepharose FF column and eluted with a linear gradient comprising the starting buffer and 200 nM NaCl. The purified soluble fraction of the stromelysin fragment is concentrated and stored at 4°C. The pellet is solubilizcd in 8M guanidine-HCl. The solution is centrifuged for 20 minutes at 20,000 rpm and the supernatant added dropwise to a folding buffer comprising 50 mM Tris-HCl (pH 7.6), 10 mM $CaCl_2$, 0.5 mM $ZnCl_2$ and the inhibitor cocktail of AEBSF, Leupeptin®, Aprotinin®, and Pepstatin® (all at concentrations of 1 μg/ml). The volume of folding buffer is ten times that of the supernatant. The mixture of supernatant and folding buffer are centrifuged at 20,000 rpm for 30 minutes. The supernatant from this centrifugation is stored at 4°C and the pellet subjected twice to the steps described above of solubilization in guanidine-HCl, refolding in buffer, and centrifugation. The final supernatants from each of the three centrifugations are combined and solid ammonium sulfate was added to the point of 20% saturation. The resulting solution thus derived from the insoluble fraction is subjected to purification on phenyl Sepharose and Q-Sepharose as described above for the soluble fraction. The purified soluble and insoluble fractions are combined to produce about 1.8 mg of purified stromelysin 81-256 fragment (SCD) per gram of original cell paste, uniformly enriched with $^{13}C$.

<u>**Preparation 2**</u>

**Preparation of Specifically $^{13}C$-Enriched Catalytic Domain of Human Stromelysin (SCD)**

SCD is expressed by culturing the BL21 (DE3) /pLysS/pETST-255-1 modified E. *coli* strain in a medium comprising $^{13}C$-enriched α-ketobutyric acid and α-keto-isovaleric acid. The methods used for preparation of the genetically-engineered strain of *E. coli,* and for expressing, isolating, and purifying the protein fragment are as described above, except for the use of U-$^{12}C$-glucose, instead of U-$^{13}C$-glucose.

<u>**Preparation 3**</u>

**Preparation of Uniformly and Specifically $^{13}C$-Enriched FKBP**

<u>A. Preparation of Specifically $^{13}C$-Enriched Val/Leu FKBP</u>

Cells transformed with a plasmid encoding for human FKBP (as described in Egan, *et al.*, *Biochemistry* 32: 1920-1927 (1993)) were grown in a 100% $D_2O$ culture medium containing $^{15}NH_4Cl$ (Cambridge Isotopes) as the sole nitrogen source (1.0 g/L) and perdeuterated glucose (1.5 g/L) as the carbon source (Cambridge Isotopes). Cells were grown at 37°C and the flask contents were shaken until an optical density of 1.0 was obtained. One hour prior to induction, 80 mg of L-valine-U-$^{13}C_5$-$^{15}N$-2,3-$d_2$ (Cambridge Isotopes) was added to the culture medium. The culture was induced with 1 mM IPTG for 12 hours.

The cells were harvested by centrifugation at 17,000 x g for 10 minutes at 4 °C and the resulting cell pellets were suspended in 50 mM phosphate buffer (pH 7.4) containing 5 mM DTT and 1 mM PMSF and mechanically lysed using a French press. The resulting lysate was centrifuged at 25,000 rpm for 30 minutes. Solid ammoniuum sulfate was added to the supernatant to the point of 40% of saturation and the centrifuged at 18,000 rpm for 5 minutes. The supernatant was dialyzed into 10 mM HEPES (pH 8.0) for 12 hours at 4°C. The resulting solution was then loaded onto a 10 mL Q-Sepharose fast flow column (Sigma) pre-equilibrated in the dialysis buffer. Fractions were collected, pooled, and concentrated using an Amicon flow cell. The solution was then dialyzed into 20 mM phosphate buffer (pH 6.5) containing 10 mM DTT and 0.01% sodium azide.

<u>B. Preparation of Specifically $^{13}C$-Enriched</u>

<u>Val/Leu/Ile FKBP</u>

Cells transformed with a plasmid encoding for human FKBP (as described in Egan, *et al.*, *Biochemistry* 32:

1920-1927 (1993)) are grown in a culture medium containing $NH_4Cl$ as the sole nitrogen source (1.0 g/L) and glucose (1.5 g/L) as the carbon source. Cells are grown at 37°C and the flask contents are shaken until an optical density of 1.0 is obtained. One hour prior to induction, 100 mg of α-keto-butyric acid and α-keto-isovaleric acid are added to the culture medium. The culture is induced with 1 mM IPTG for 12 hours. Expression, isolation, and purification of the expressed protein are as described above.

C. Preparation of Uniformly $^{13}C$-Enriched FKBP

Cells transformed with a plasmid encoding for human FKBP (as described in Egan, *et al., Biochemistry* 32: 1920-1927 (1993)) were grown in a culture medium containing $^{15}NH_4Cl$ (Cambridge Isotopes) as the sole nitrogen source (1.0 g/L) and uniformly $^{13}C$-enriched glucose (1.5 g/L) as the carbon source (Cambridge Isotopes). Expression, isolation, and purification of the expressed protein are as described above.

## Example 1

### Screening for Ligands Using $^{13}C/^{1}H$ NMR Correlation Spectra Using Uniformly $^{13}C$-Enriched FKBP

Uniformly $^{13}C$-enriched FKBP was prepared in accordance with the procedures detailed above. The protein solutions used in the screening assay contained the uniformly $^{13}C$-enriched FKBP (0.2 mM) and sodium azide (0.05%) in an $H_2O/D_2O$ (9/1) phosphate buffered solution (20 mM, pH 6.5).

Two-dimensional $^{13}C/^{1}H$ NMR spectra were generated at 30°C on a Bruker DRX500 NMR spectrometer equipped with a triple resonance probe and Bruker sample changer. The $^{13}C/^{1}H$ HSQC spectra were acquired as 64 x 1024 complex points using sweep widths of 3771 Hz ($^{13}C$, $t_1$) and 8333 Hz ($^{1}H$, $t_2$). A delay of 1 second between scans and 8 scans per free induction decay (fid) were employed in the data collection. All NMR spectra were processed and analyzed on Silicon Graphics computers.

A first two-dimensional $^{13}C/^{1}H$ NMR correlation spectrum was acquired for the $^{13}C$-labeled FKBP target molecule as described above. The FKBP target was then exposed to a library mixture of test compounds. Stock solutions of the compounds were made at 100 mM and 1 M. In addition, a combination library was prepared which contained 8-10 compounds per sample at a concentration of 100 mM for each compound.

The pH of the 1 M stock solution was adjusted with acetic acid and ethanolamine so that no pH change was observed upon a 1/10 dilution with a 100 mM phosphate buffered solution (pH 7.0). It is important to adjust the pH, because small changes in pH can alter the chemical shifts of the biomolecules and complicate the interpretation of the NMR data.

The compounds in the library were selected on the basis of size (molecular weight = 100-300) and molecular diversity. The molecules in the collection had different shapes (for example, flat aromatic rings(s), puckered aliphatic rings(s), straight and branched chain aliphatics with single, double, or triple bonds) and diverse functional groups (for example, carboxylic acids, esters, ethers, amines, aldehydes, ketones, and various heterocyclic rings) to maximize the possibility of discovering compounds that interact with widely diverse binding sites.

The NMR samples were prepared by adding 1.25 μl of the dimethyl sulfoxide stock solution of the compound mixtures that contained each compound at a concentration of 100 mM to 0.5 ml $H_2O/D_2O$ (9/1) buffered solution of the uniformly $^{13}C$-labeled protein. The final concentration of each of the compounds in the NMR sample was about 0.25 mM.

In the screening experiment, one compound, 2-phenylimidazole, was found to bind to FKBP. Figure 1 shows the $^{13}C/^{1}H$ correlation spectrum of uniformly $^{13}C$-labeled FKBP. The spectrum (128 complex points, 4 scans/fid) was acquired on a 0.1 mM sample of FKBP in 20 mM phosphate (pH 6.5), 0.01% sodium azide and 10% deuterium oxide ($D_2O$).

Figure 1 shows a $^{13}C/^{1}H$ correlation spectrum of uniformly $^{13}C$-labeled FKBP. The spectrum (128 complex points, 4 scans/fid) was acquired on a 0.1 mM sample of FKBP in 20 mM phosphate (pH 6.5), 0.01% sodium azide and 10% deuterium oxide ($D_2O$). Figure 2 shows the methyl regions of $^{13}C/^{1}H$ correlation spectra (64 complex points, 8 scans/fid) of uniformly $^{13}C$-labeled FKBP (0.2 mM) before (thin multiple contours) and after (thick single contours) the addition of 2-phenylimidazole (0.25 mM). The changes in chemical shifts at aminoacyl residues $Leu^{97}$, $Val^{55}$, $Ile^{56}$ and $Ile^{90}$ are indicated.

Figure 3 shows $^{13}C/^{1}H$ correlation spectra (48 complex points, 8 scans/fid) of FKBP selectively $^{13}C/^{15}N/^{2}H$ labeled at valinyl and leucyl residues before (thin multiple contours) and after (thick single contours) addition of 2-phenylimidazole (0.25 mM). All other conditions are the same as those employed in generating the spectra illustrated in Figure 1. Selected residues that show significant changes upon binding are indicated. Again, changes in the chemical shift values indicate that binding is occurring at or near the $Leu^{97}$ and $Val^{55}$ residues.

Figure 4 shows $^{13}C/^{1}H$ correlation spectra of FKBP selectively $^{13}C$ labeled at alanyl residues before (thin multiple contours) and after (thick single contours) addition of 2-phenylimidazole (0.25 mM). The super-position of the chemical shift values before (light multiple contours) and after (heavy single contour) addition of the ligand indicate that

none of the alanyl residues are involved in the binding.

Figure 5 shows a "stick model" depiction of the three-dimensional structure of FKBP. Selected residues are numbered for aid in visualization. The aminoacyl residues involved in the binding site in the protein have been shown in bold.

**Claims**

1. A method of detecting binding between a putative ligand and a pre-selected, $^{13}C$-enriched target molecule which comprises:

   a) generating a first two-dimensional $^{13}C/^{1}H$ NMR correlation spectrum of said target molecule;
   b) forming a mixture of said target molecule with at least one putative ligand compound;
   c) generating a second two-dimensional $^{13}C/^{1}H$ NMR correlation spectrum of the mixture of step b); and
   d) comparing the first and the second spectra;

   wherein said target molecule is prepared by culturing a transformed cell line which contains an expression vector containing a polynucleotide encoding said target molecule in a medium containing a biosynthetic precursor of an amino acid selected from the group consisting of 4-($^{13}C$)-$\alpha$ keto butyric acid and 4-($^{13}C$)-3-($^{13}C$)-$\alpha$ keto methylbutyric acid.

2. A method of screening a mixture of compounds for binding to a pre-selected $^{13}C$-enriched target molecule which comprises:

   a) generating a first two-dimensional $^{13}C/^{1}H$ NMR correlation spectrum of said target molecule;
   b) forming a mixture of said target molecule with at least one putative ligand compound;
   c) generating a second two-dimensional $^{13}C/^{1}H$ NMR correlation spectrum of the mixture of step b); and
   d) comparing the first and the second spectra;

   wherein said target molecule is prepared by culturing a transformed cell line which contains an expression vector containing a polynucleotide encoding said target molecule in a medium containing a biosynthetic precursor of an amino acid selected from the group consisting of 4-($^{13}C$)-$\alpha$ keto butyric acid and 4-($^{13}C$)-3-($^{13}C$)-$\alpha$ keto methylbutyric acid.

**Patentansprüche**

1. Ein Verfahren zur Detektion der Bindung zwischen einem vermeintlichen Liganden und einem vorgewählten $^{13}C$-angereicherten Zielmolekül, das folgendes umfaßt:

   a) Erzeugen eines ersten zweidimensionalen $^{13}C/^{1}H$ NMR Korrelationsspektrums des Zielmoleküls;
   b) Bilden einer Mischung des Zielmoleküls mit mindestens einer vermeintlichen Ligandenverbindung;
   c) Erzeugen eines zweiten zweidimensionalen $^{13}C/^{1}H$ NMR Korrelationsspektrums der Mischung von Schritt b); und
   d) Vergleichen des ersten und des zweiten Spektrums;

   worin das Zielmolekül hergestellt wird durch Kultivieren einer transformierten Zell-Linie, welche einen Expressionsvektor enthält, der ein Polynukleotid enthält, das das Zielmolekül kodiert, in einem Medium, welches einen biosynthetischen Vorläufer einer Aminosäure enthält, die gewählt ist aus der Gruppe bestehend aus 4-($^{13}C$)-$\alpha$ Ketobuttersäure und 4-($^{13}C$)-3-($^{13}C$)-$\alpha$ Ketomethylbuttersäure.

**2.** Ein Verfahren zum Screenen einer Mischung aus Verbindungen für die Bindung an ein vorgewähltes $^{13}C$-angereichertes Zielmolekül, das folgendes umfaßt:

a) Erzeugen eines ersten zweidimensionalen $^{13}C/^{1}H$ NMR Korrelationsspektrums des Zielmoleküls;
b) Bilden einer Mischung des Zielmoleküls mit mindestens einer vermeintlichen Ligandenverbindung;
c) Erzeugen eines zweiten zweidimensionalen $^{13}C/^{1}H$ NMR Korrelationsspektrums der Mischung von Schritt b); und
d) Vergleichen des ersten und des zweiten Spektrums;

worin das Zielmolekül hergestellt wird durch Kultivieren einer transformierten Zell-Linie, welche einen Expressionsvektor enthält, der ein Polynukleotid enthält, das das Zielmolekül kodiert, in einem Medium, das einen biosynthetischen Vorläufer einer Aminosäure enthält, die gewählt ist aus der Gruppe bestehend aus 4-($^{13}C$)-$\alpha$ Ketobuttersäure und 4-($^{13}C$)-3-($^{13}C$) - $\alpha$ Ketomethylbuttersäure.

## Revendications

**1.** Procédé de détection de la liaison entre un ligand putatif et une molécule cible présélectionnée, enrichie en $^{13}C$, qui comprend:

a) la production d'un premier spectre de corrélation de RMN de $^{13}C/^{1}H$ bidimensionnel de ladite molécule cible;
b) la formation d'un mélange de ladite molécule cible avec au moins un composé ligand putatif;
c) la production d'un deuxième spectre de corrélation de RMN de $^{13}C/^{1}H$ bidimensionnel du mélange de l'étape b); et
d) la comparaison du premier et du deuxième spectre;

ladite molécule cible étant préparée par culture d'une lignée cellulaire transformée qui contient un vecteur d'expression contenant un polynucléotide codant pour ladite molécule cible dans un milieu contenant un précurseur biosynthétique d'un aminoacide choisi dans le groupe constitué par l'acide 4-($^{13}C$)-$\alpha$-cétobutyrique et l'acide 4-($^{13}C$)-3-($^{13}C$)-$\alpha$-cétométhylbutyrique.

**2.** Procédé de criblage d'un mélange de composés pour la liaison à une molécule cible enrichie en $^{13}C$ présélectionnée, qui comprend:

a) la production d'un premier spectre de corrélation de RMN de $^{13}C/^{1}H$ bidimensionnel de ladite molécule cible;
b) la formation d'un mélange de ladite molécule cible avec au moins un composé ligand putatif;
c) la production d'un deuxième spectre de corrélation de RMN de $^{13}C/^{1}H$ bidimensionnel du mélange de l'étape b); et
d) la comparaison du premier et du deuxième spectre;

ladite molécule cible étant préparée par culture d'une lignée cellulaire transformée qui contient un vecteur d'expression contenant un polynucléotide codant pour ladite molécule cible dans un milieu contenant un précurseur biosynthétique d'un aminoacide choisi dans le groupe constitué par l'acide 4-($^{13}C$)-$\alpha$-cétobutyrique et l'acide 4-($^{13}C$)-3-($^{13}C$)-$\alpha$-cétométhylbutyrique.

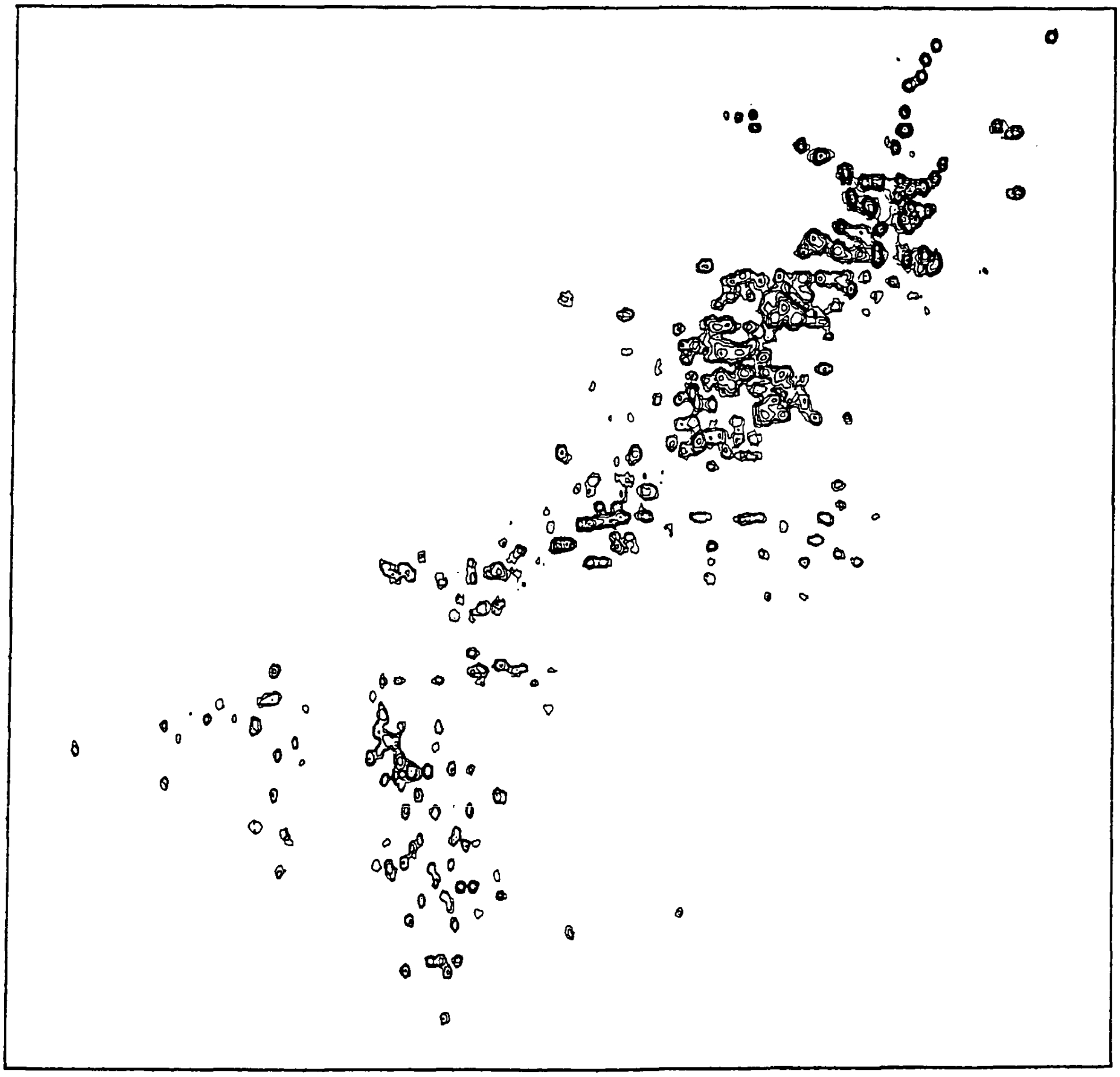

FIG. 1

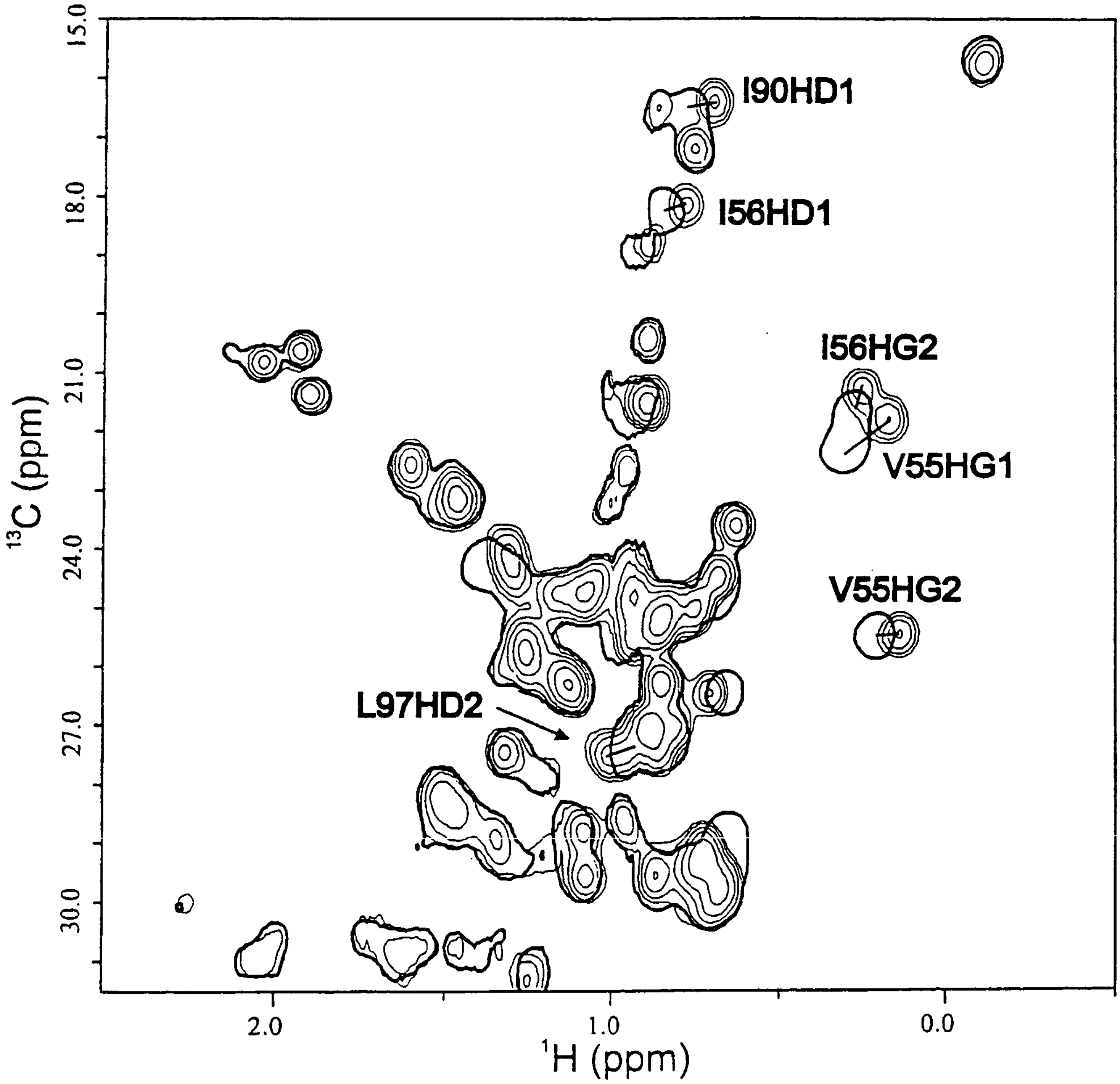
15.0
18.0
21.0
24.0
27.0
30.0
13C (ppm)
I90HD1
I56HD1
I56HG2
V55HG1
V55HG2
L97HD2
2.0
1.0
0.0
1H (ppm)

FIG.2

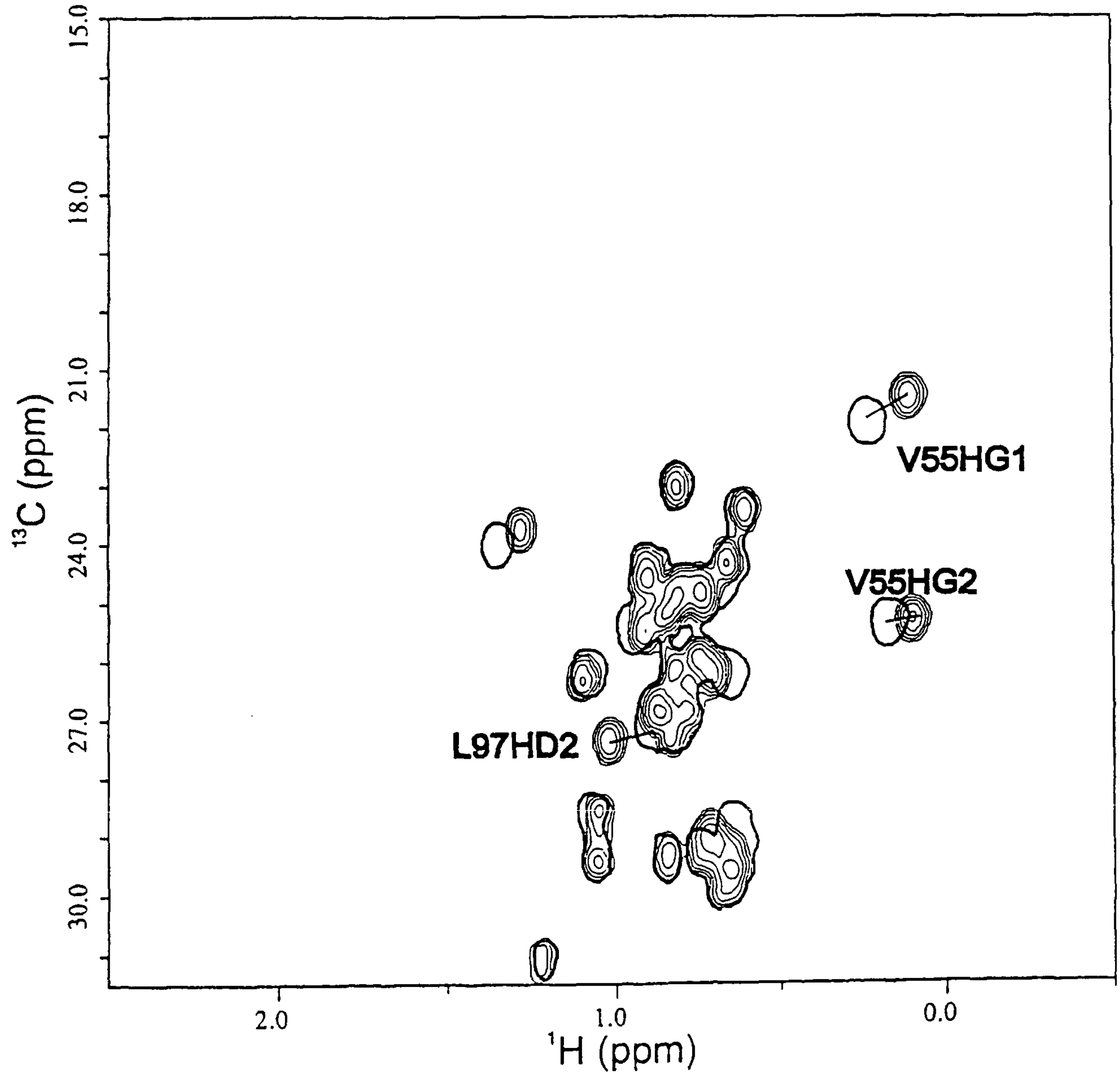
15.0
18.0
21.0
24.0
27.0
30.0
$^{13}C$ (ppm)
V55HG1
V55HG2
L97HD2
2.0
1.0
0.0
$^{1}H$ (ppm)

FIG.3

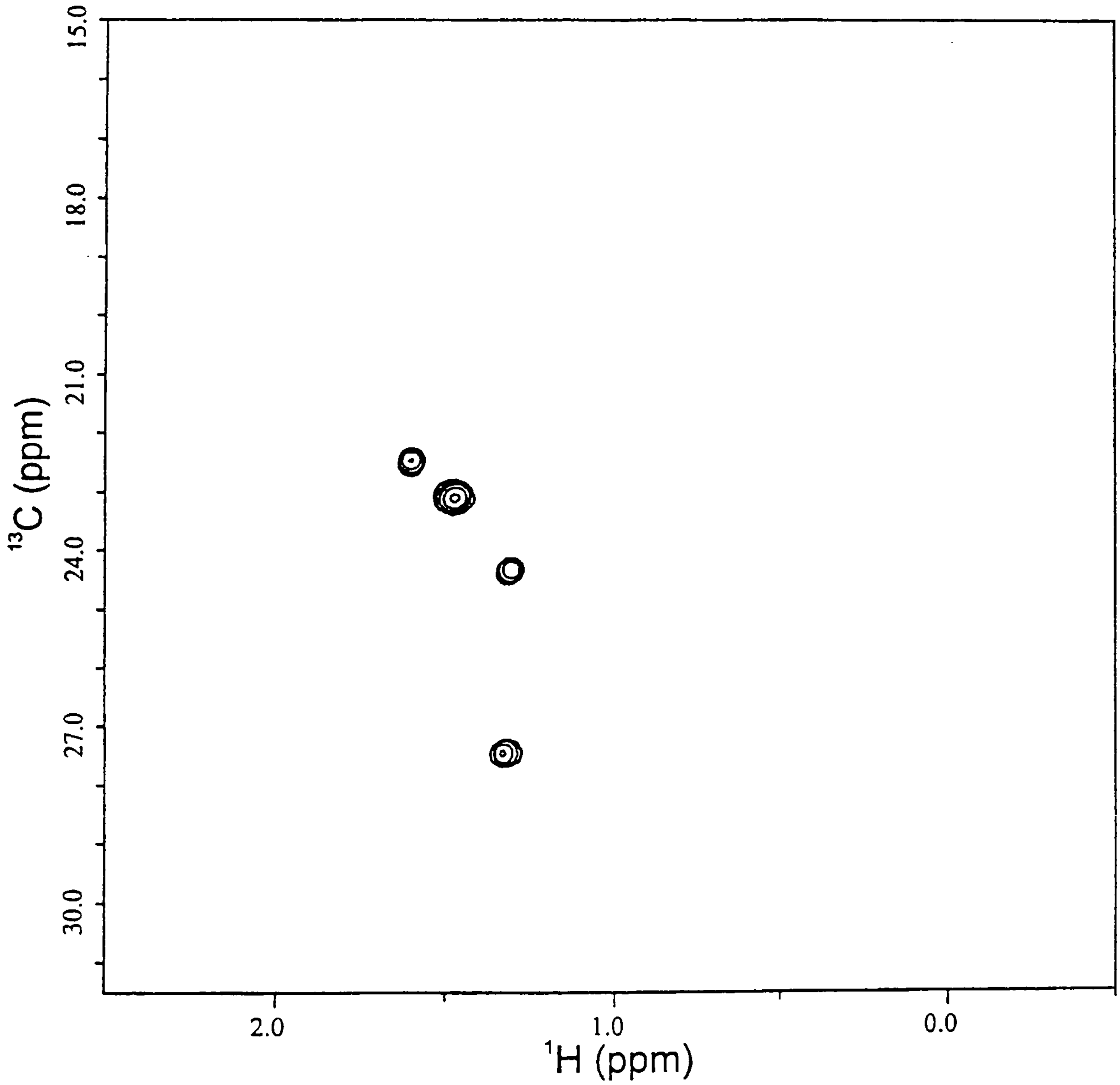


FIG.4

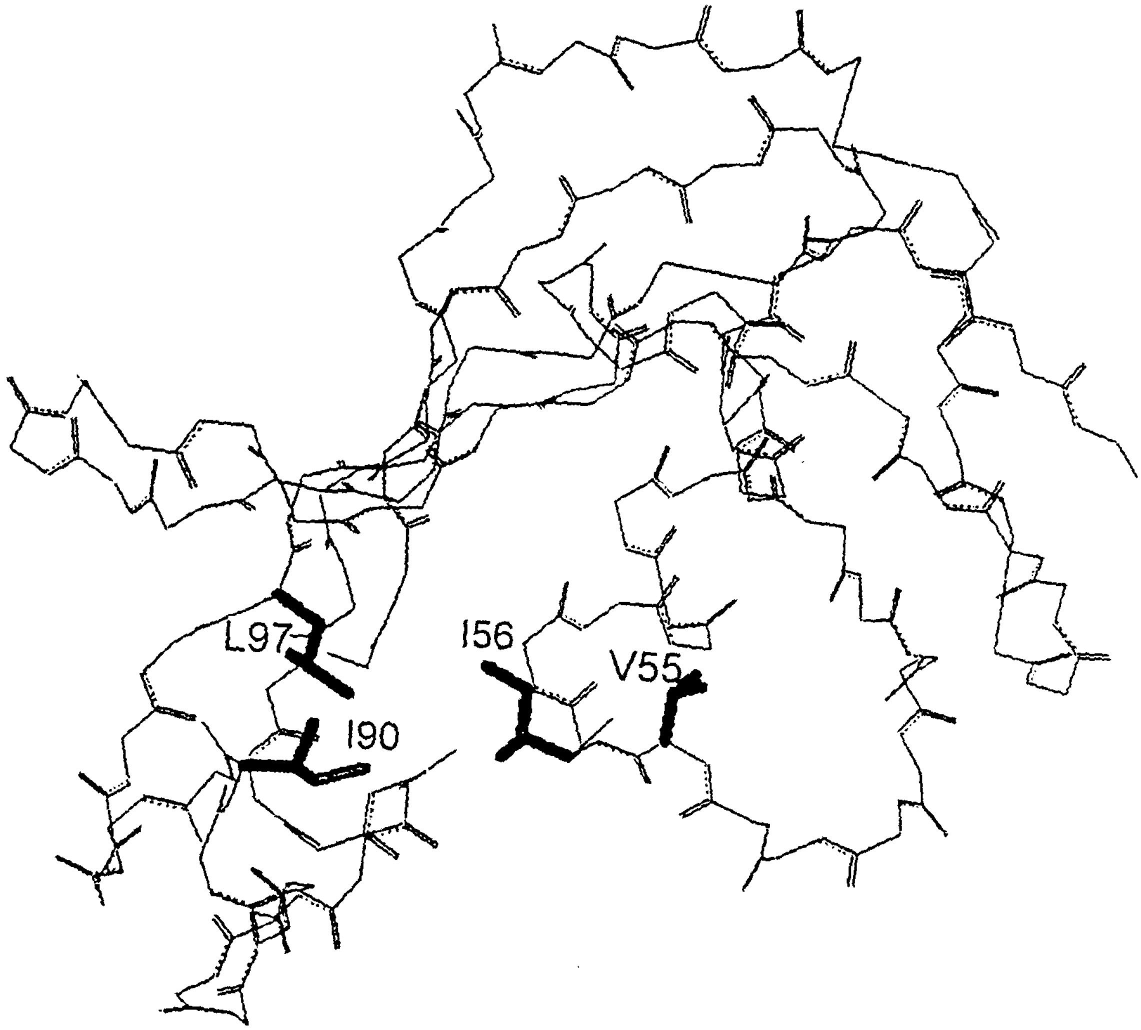


FIG.5